# EUROPÄISCHE PATENTSCHRIFT

(11) **EP 1 304 097 B1**
(45) Veröffentlichungstag und Bekanntmachung des Hinweises auf die Patenterteilung: **15.09.2004**
(21) Anmeldenummer: 02023031.4
(22) Anmeldetag: 16.10.2002
(51) Int. Cl.: A61H 35/04, A61M 3/02

(54) **Nasendusche**
Nasal irrigator
Douche nasale

(30) Priorität: 19.10.2001 DE 10151676
(43) Veröffentlichungstag der Anmeldung: 23.04.2003
(73) Patentinhaber: HEXAL AG, 83607 Holzkirchen (DE)
(72) Erfinder: Philipps, Tom, Prof., 64297 Darmstadt (DE); Weber, Klaus, 56291 Leiningen (DE); Hirsch, Olaf, 56068 Koblenz (DE); Karow, Eva-Maria, Dr., 56412 Niedererbach (DE)
(74) Vertreter: Philipp, Matthias, Dr.

(56) Entgegenhaltungen:
- DE-A- 3 332 723
- DE-A- 3 929 964
- PATENT ABSTRACTS OF JAPAN vol. 1998, no. 05, 30. April 1998 (1998-04-30) -& JP 10 017039 A (KANEBO LTD;KEY TRANDING CO LTD), 20. Januar 1998 (1998-01-20)

## Beschreibung

Die Erfindung betrifft eine Nasendusche mit einem Behälter für Spülflüssigkeit, der eine Austrittsöffnung aufweist, mit einem im Bereich der Austrittsöffnung beweglich gehaltenen Ventilkörper mit einem an diesem angesetzten Rohr, wobei unterschiedliche Schwenkpositionen des Ventilkörpers in Zusammenwirken mit der Austrittsöffnung eine Offenstellung, in der das Rohr mit dem Behälter verbunden ist, und eine Schließstellung, in der der Behälter verschlossen, festlegen, wie sie aus der DE 39 29 964 C2 bekannt ist.

Die bekannte Nasendusche hat sich an sich in der Praxis bestens bewährt, wobei es sich allerdings im Gebrauch gelegentlich als unzweckmäßig herausgestellt hat, die Dusche nach Befüllung mit Spülflüssigkeit stets in der Hand halten zu müssen, da andernfalls die Flüssigkeit ausläuft. Ferner konnte es zu Beschädigungen des vom Behälter abstehenden Rohrs und/oder des beweglichen Ventilkörpers kommen.

Eine Aufgabe der Erfindung besteht daher darin, die bekannte Nasendusche dahingehend weiterzuentwickeln, daß sie im Gebrauch zweckmäßiger handhabbar ist und eine Beschädigung des Ventilkörpers bzw. des daran angesetzten Rohrs im wesentlichen ausgeschlossen werden kann.

Diese Aufgabe wird erfindungsgemäß durch eine Nasendusche nach Anspruch 1 gelöst. Zweckmäßige Weiterbildungen der Erfindung sind in den Unteransprüchen aufgezeigt.

Die Erfindung wird nachfolgend anhand eines Ausführungsbeispiels erläutert, wobei auf eine Zeichnung Bezug genommen ist, in der
Fig. 1 eine Querschnittsansicht der erfindungsgemäßen Nasendusche in Schließstellung zeigt;
Fig. 2 eine Ansicht entsprechend Fig. 1 in Offenstellung zeigt;
Fig. 3 eine Ansicht entsprechend Fig. 1 und 2 in Belüftungsstellung zeigt;
Fig. 4 eine auseinandergezogene, perspektivische Ansicht von Einzelteilen der erfindungsgemäßen Dusche zeigt;
Fig. 5 eine perspektivische Ansicht des Ventilkörpers mit angesetztem Rohr zeigt;
Fig. 6 eine perspektivische Ansicht aus einem anderen Blickwinkel von Ventilkörper und Rohr zeigt;
Fig. 7 eine vergrößerte perspektivische Ansicht des den Ventilkörper aufnehmenden Bereichs der Nasendusche zeigt; und
Fig. 8 den Bereich nach Fig. 7 aus einer anderen Richtung in einer teilweise abgeschnitten Ansicht zeigt.

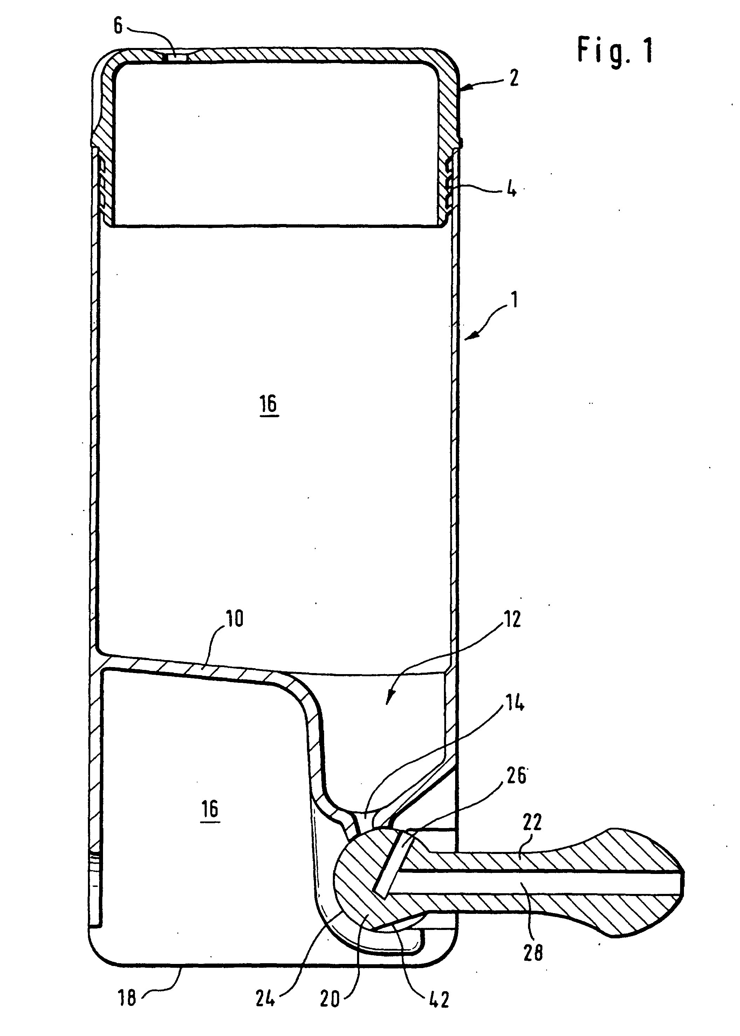

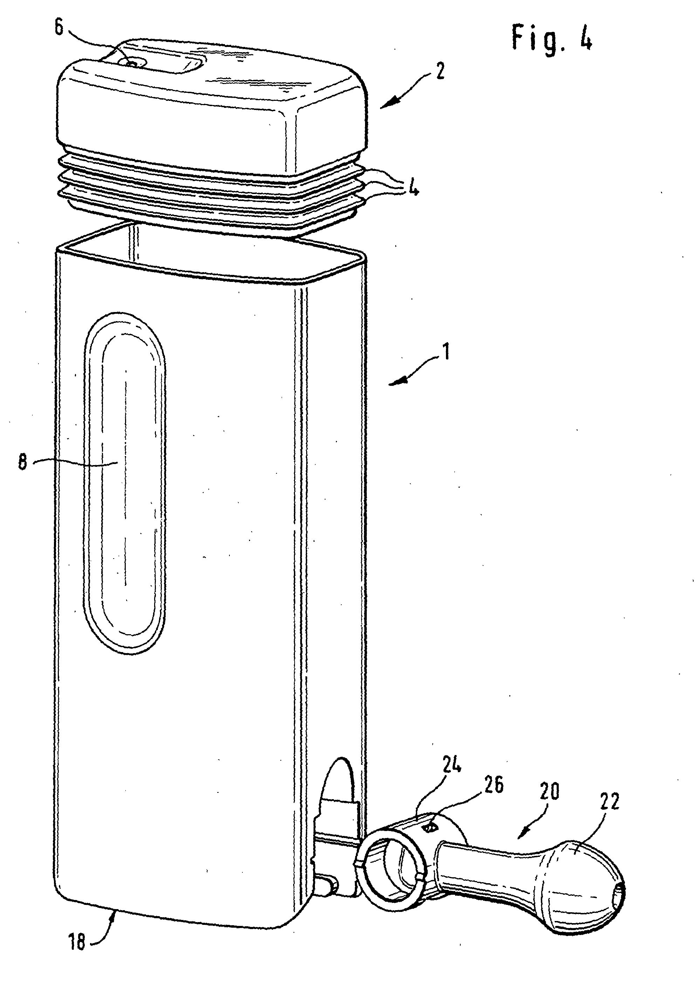

Zunächst sei auf Fig. 1 bis 4 Bezug genommen, die Aufbau und Funktionsweise der erfindungsgemäßen Nasendusche zeigen. Die Nasendusche ist in ihrer aufrecht stehenden bzw. senkrechten Gebrauchsstellung dargestellt. Ein im wesentlichen zylindrischer Behälter 1 ist mit einem abnehmbaren Deckel 2, der umlaufende Dichtlippen 4 aufweist, luft- und flüssigkeitsdicht verschlossen, wobei der Deckel 2 eine mit einem Finger vorübergehend verschließbare bzw. freizugebende Dosieröffnung 6 aufweist. Es kann vorgesehen sein, daß der Deckel 2 mittels einer umlaufenden Klemmnut mit einer Dicht- und Halterungswulst des Behälters zusammenwirkt, so daß er sich auch dann nicht von dem Behälter löst bzw. auch dann noch dicht schließt, wenn ein Innendruck in dem Behälter auftritt (Dosierung unter Druck, s.u.). An äußeren Seitenwänden 16 des Behälters eingeformte Griffmulden 8 (Fig. 4) erleichtern das Halten der Dusche und das gleichzeitige Verschließen bzw. Öffnen der Dosieröffnung 6.

Der Behälter ist innen etwa in seinem unteren Drittel mit einer Bodenwand 10 abgeschlossen, die eine Vertiefung 12 mit einer im untersten Bereich der Vertiefung angeordneten Austrittsöffnung 14 bildet. Die Seitenwände 16 des Behälters sind über den Bereich der Bodenwand 10 hinweg nach unten verlängert, so daß Standflächen 18 zum Abstellen des Behälters bzw. der Nasendusche in der dargestellten (vertikalen) Gebrauchsstellung gebildet werden.

Ein im wesentlichen zylindrischer Ventilkörper 20 mit einem von diesem ausgehenden Rohr 22 ist benachbart zu der Austrittsöffnung 14 schwenkbar gelagert, so daß die in Fig. 1 bis 3 dargestellten unterschiedlichen Schwenkstellungen eingenommen werden können.

Fig. 1 zeigt die Schließstellung des Ventilkörpers, in der die Austrittsöffnung 14 des Behälters durch eine zylindrische Außenfläche 24 des Ventilkörpers 20 verschlossen ist.

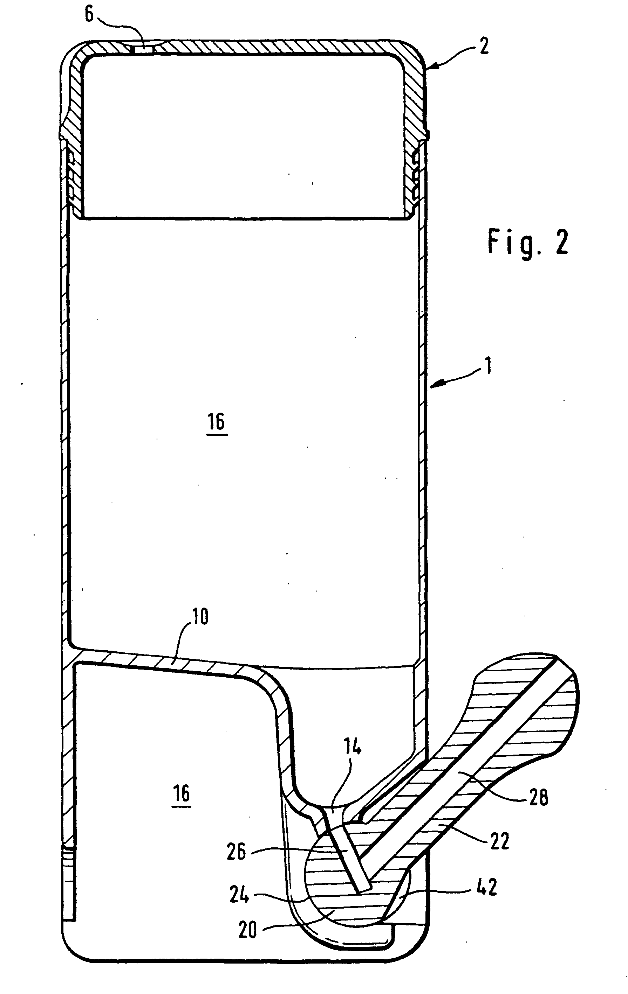

Fig. 2 zeigt die Benutzungs- oder Offenstellung, wobei eine im wesentlichen radial in dem Ventilkörper 20 angeordneter Verbindungskanal 26 mit der Austrittsöffnung 14 in Verbindung steht, während ein durch den Ventilkörper und das Rohr 22 verlaufender Abgabekanal 28an den Verbindungskanal 26 anschließt, so daß eine innerhalb des Behälters befindliche Flüssigkeit durch den Abgabekanal 28 austreten kann.

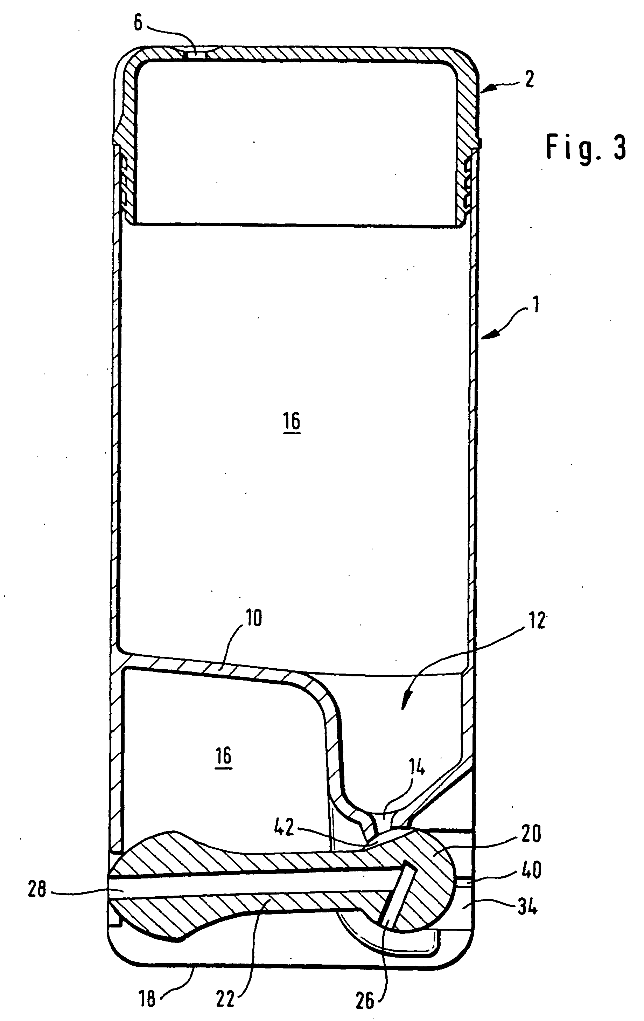

Fig. 3 zeigt eine Belüftungsstellung des Ventilkörpers 20, in der dieser soweit verschwenkt ist. daß das damit verbundene Rohr 22 vollständig zwischen den verlängerten Seitenwänden 16 liegt und somit nicht mehr über die zylindrische oder angenähert quaderförmige Außenkontur des Behälters 1 übersteht, sondern von dieser abgedeckt ist.

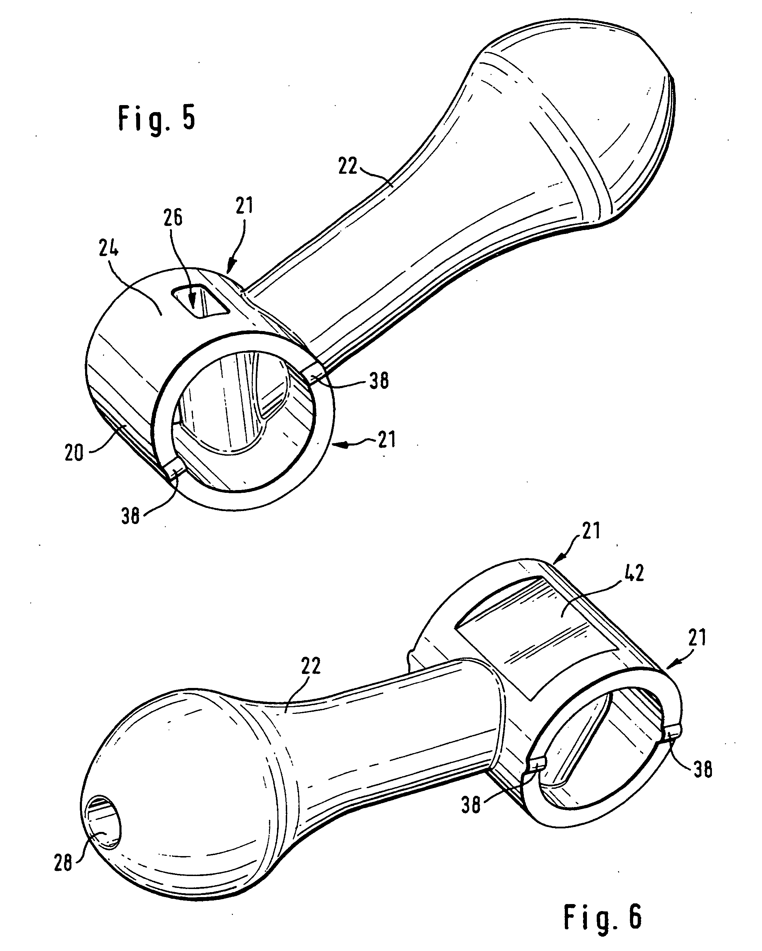

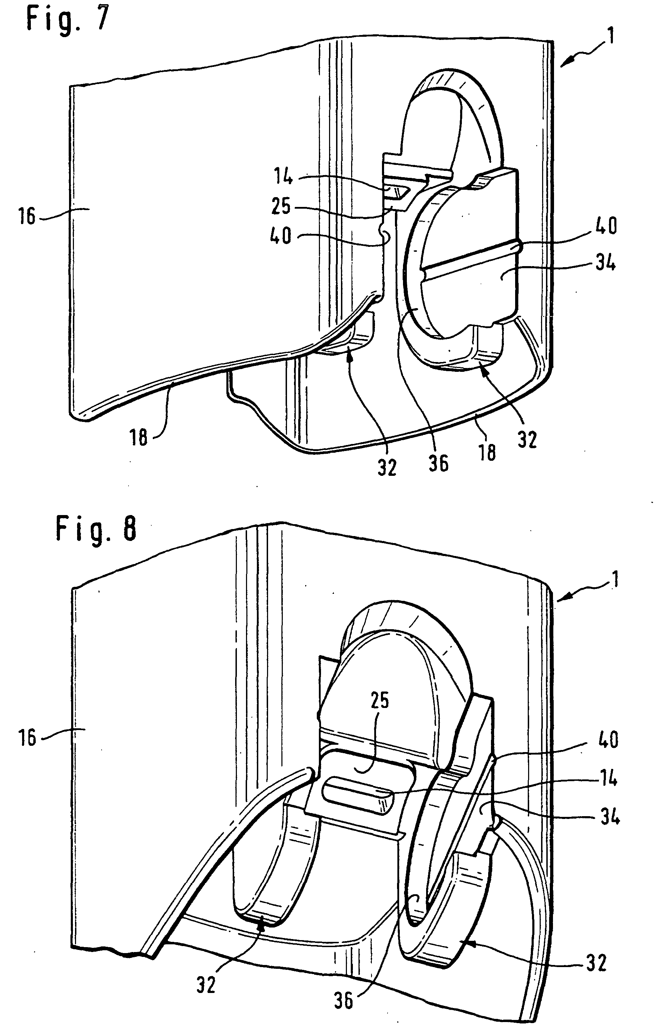

Fig. 5 und 6 zeigen vergrößerte Ansichten des Ventilkörpers 20 sowie des damit verbundenen Rohrs 22, woraus die hohlzylindrische Grundform des Ventilkörpers ersichtlich ist. Der Verbindungskanal 26 hat rechteckigen Querschnitt und entspricht in Form und Größe in etwa der Austrittsöffnung 14 (Fig. 7 und 8) bzw. der um diese herum ausgebildeten, mit der Dichtfläche 24 zusammenwirkenden Anlagefläche 25.

Am unteren Bereich des Behälters sind an der Bodenwand 10 und den Seitenwänden 16 im wesentlichen ebene Lagerlappen 32 ausgebildet, die in seitlicher bzw. axialer Richtung (bezogen auf die Schwenkachse des Ventilkörpers) ebene Begrenzungswände 34 sowie C-förmige, in Umfangsrichtung über mehr als 180° Umfangswinkel verlaufende Lagerstege 36 aufweisen. Diese Ausbildung ermöglicht es, daß der Ventilkörper 20 gegen die elastisch geringfügig verformbaren Lagerlappen 32 bzw. die Lagerstege 36 eingerastet und, in umgekehrter Richtung, herausgenommen werden kann, so daß der Ventilkörper zerstörungsfrei einsetzbar und herausnehmbar ist.

Alternativ könnten die der Lagerung dienenden, seitlichen zylindrischen Endabschnitte des Ventilkörpers 20 sowie die Lagerstege 36 (bzw. der Ventilkörper insgesamt) asymmetrisch ausgebildet sein, z.B. links und rechts unterschiedliche Durchmesser haben, damit sichergestellt ist, daß der Ventilkörper nur in der korrekten Orientierung (und nicht um 180° verdreht) eingesetzt werden kann.

Wie Fig. 5 bis 7 ferner zeigen, sind die hohlzylindrischen Endabschnitte bzw. Lagerungsansätze 21 des Ventilkörpers 20 mit Rastnasen 38 versehen, die in Schließstellung (Fig. 1) und Belüftungsstellung (Fig. 3) mit entsprechenden Rastnuten 40 in den Begrenzungswänden 34 der Lagerlappen 32 zusammenwirken, so daß der Ventilkörper 20 in den genannten Stellungen arretiert gehalten wird und ein versehentliches Verschwenken in die Öffnungsstellung verhindert wird. Die Rastnasen 38 und Rastnuten 40 können alternativ auch weggelassen werden oder durch anders ausgebildete Rastmittel ersetzt werden.

Wie Fig. 3 und 6 weiter zeigen, ist der Ventilkörper 20 mit einer schrägen, tangentialen Abflachung oder Restentleerungs-Aussparung 42 versehen, die in Belüftungsstellung (Fig. 3) der Abgabeöffnung 14 gegenübersteht. Dadurch wird erreicht, daß der Behälter 1 in Belüftungsstellung an beiden Enden, nämlich an der Dosierungsöffnung 6 einerseits und der Abgabeöffnung 14 andererseits, offen ist und mit der Umgebungsluft in Verbindung steht, so daß eine Restentleerung und Belüftung sichergestellt ist.

Zum Gebrauch wird die Nasendusche in Verschlußstellung (Fig. 1) gebracht, der Deckel 2 abgenommen, eine gewünschte Spülflüssigkeit eingefüllt und der Deckel wieder aufgesetzt. Der Benutzer nimmt das Gerät in der in den Zeichnungsfiguren dargestellten, vertikalen Orientierung in eine Hand und verschließt, z.B. mit dem Zeigefinger, die Dosierungsöffnung 6. Anschließend wird der Ventilkörper (ganz oder teilweise) in Öffnungsstellung (Fig. 2) gebracht, und das Gerät ist nach Ansetzen an die Nase einsatzbereit, wobei eine Dosierung der Spülflüssigkeit durch (teilweises) Öffnen der Dosierungsöffnung 6 erfolgt, so daß die Spülflüssigkeit allein durch Schwerkraftwirkung (hydrostatischer Druck) aus dem Rohr 22 austritt. Wenn gewünscht, kann der Druck durch Schließen der Dosierungsöffnung 6 und Zusammendrücken des Behälters 1 (Druck auf die Seitenwände 16) erhöht werden.

Nach Gebrauch bringt man den Ventilkörper in Belüftungsstellung (Fig. 3), wodurch die Abgabeöffnung 14 aufgrund der Restentleerungs-Aussparung 42 offen ist und ggf. im Behälter 1 befindliche Reste an Spülflüssigkeit frei austreten können. In der Belüftungsstellung befinden sich sämtliche mit Spülflüssigkeit in Berührung kommenden Flächen bzw. Kanäle des Geräts in Verbindung mit der Umgebung, nämlich der Behälter 1, die Austrittsöffnung 14, der Abgabekanal 28 sowie die Verbindungsöffnung 26.

Zum Zwecke einer gründlichen Reinigung des Geräts kann der Ventilkörper 20, wie bereits erwähnt, beschädigungsfrei aus seiner Lagerung herausgenommen und wieder eingesetzt werden. Wichtig ist hierbei, daß die Dichtfunktion (Ventilkörper gegenüber Abgabeöffnung) von der Lagerungsfunktion (Ventilkörper an Behälter) konstruktiv getrennt ist, so daß auch nach wiederholtem Herausnehmen und Wiedereinsetzen des Ventilkörpers die Dichtfunktion nicht beeinträchtigt ist.

Ein weiterer Vorteil besteht darin, daß das Gerät auf den Standflächen 18 am unteren Ende der Seitenwände 16 in allen Stellungen des Ventilkörpers 20 (Fig. 1 bis 3) abstellbar ist, insbesondere in Schließ- und Belüftungsstellung, so daß die Handhabung verbessert ist. Die Möglichkeit, den Ventilkörper in Belüftungsstellung zu bringen, bietet den Vorteil, daß das Rohr 22 vor Verunreinigungen und Beschädigungen geschützt ist, wobei die Restentleerungs-Aussparung 42 gleichzeitig für Entleerung und Belüftung sorgt.

### Bezugszeichenliste

- 1: Behälter
- 2: Deckel
- 4: Dichtungslippe
- 6: Dosierungsöffnung
- 8: Griffmulde
- 10: Bodenwand
- 23: Vertiefung
- 14: Abgabeöffnung
- 16: Seitenwand
- 18: Standfläche
- 20: Ventilkörper
- 21: Lagerungsansatz
- 22: Rohr
- 24: Außenfläche (Dichtfläche)
- 25: Anlagefläche
- 26: Verbindungskanal
- 28: Abgabekanal
- 32: Lagerlappen
- 34: Begrenzungswand
- 36: Lagerungssteg
- 38: Rastnase
- 40: Rastnut
- 42: Restentleerungs-Aussparung

## Patentansprüche

1. Nasendusche mit einem Behälter (1) für Spülflüssigkeit, der eine Austrittsöffnung (14) aufweist, mit einem im Bereich der Austrittsöffnung (14) beweglich gehaltenen Ventilkörper (20) mit einem an diesem angesetzten Rohr (22), wobei unterschiedliche Schwenkpositionen des Ventilkörpers (20) in Zusammenwirken mit der Austrittsöffnung (14) eine Offenstellung (Fig. 2), in der das Rohr (22) mit dem Behälter (1) verbunden ist, und eine Schließstellung (Fig. 1), in der der Behälter (1) verschlossen ist, festlegen, **dadurch gekennzeichnet, daß** der Ventilkörper (20) in eine von Offen- und Schließstellung verschiedene Belüftungsstellung (Fig. 3), in der der Behälter restentleerbar und belüftet ist, schwenkbar ist.

2. Dusche nach Anspruch 1, **dadurch gekennzeichnet, daß** der Behälter eine Ventilkörper (20) und Rohr (22) in der Belüftungsstellung übergreifende bzw. abdeckende Außenkontur aufweist, an der Standflächen (18) ausgebildet sind, auf denen die Dusche abstellbar ist.

3. Dusche nach Anspruch 1 oder 2, **dadurch gekennzeichnet, daß** der Ventilkörper (20) eine Restentleerungs-Aussparung (42) aufweist, die in Belüftungsstellung mit der Austrittsöffnung (14) kommuniziert.

4. Dusche nach einem der Ansprüche 1 bis 3, **dadurch gekennzeichnet, daß** der Ventilkörper (20) eine gekrümmte Dichtfläche (24) aufweist, die in Offen- und Schließstellung mit der Austrittsöffnung (14) zusammenwirkt, wobei in Offenstellung ein mit dem Rohr (22) verbundener und in die Dichtfläche (24) mündender Verbindungskanal (26) mit der Austrittsöffnung (14) kommuniziert.

5. Dusche nach Anspruch 4, **dadurch gekennzeichnet, daß** der Behälter außenseitig im Bereich der Abgabeöffnung (14) eine der Dichtfläche (24) entsprechend gekrümmte Anlagefläche (25) aufweist.

6. Dusche nach einem der vorangehenden Ansprüche, **dadurch gekennzeichnet, daß** dem Ventilkörper (20) Rastelemente (38) zugeordnet sind, die in mindestens einer Stellung, insbesondere Schließ- und/oder Belüftungsstellung, mit entsprechenden Rastelementen (40) des Behälters zusammenwirken und den Ventilkörper (20) gegen unbeabsichtigtes Verschwenken sichern.

7. Dusche nach einem der vorangehenden Ansprüche, **dadurch gekennzeichnet, daß** der Ventilkörper (20) durch Verschwenken um etwa 30° bis etwa 60°, insbesondere etwa 45°, aus der Schließstellung in die Offenstellung überführbar ist.

8. Dusche nach einem der vorangehenden Ansprüche, **dadurch gekennzeichnet, daß** der Ventilkörper (20) zylinderförmig ausgebildet und an seinen Endabschnitten mit Lagerungsansätzen (21) versehen ist.

9. Dusche nach einem der vorangehenden Ansprüche, **dadurch gekennzeichnet, daß** der Ventilkörper (20) zylindrische Lagerungsansätze (21) zur Aufnahme in entsprechenden Lagerungsausnehmungen (34, 36) des Behälters (1) aufweist.

10. Dusche nach Anspruch 9, **dadurch gekennzeichnet, daß** die Lagerungsansätze (21) und Lagerungsausnehmungen (34, 36) asymmetrisch ausgebildet sind.

11. Dusche nach einem der Ansprüche 8 bis 10, **dadurch gekennzeichnet, daß** die Restentleerungs-Aussparung (42) als Abflachung des zylinderförmigen Ventilkörpers (20) ausgebildet ist.

12. Dusche nach einem der vorangehenden Ansprüche, **dadurch gekennzeichnet, daß** der Ventilkörper zerstörungsfrei aus dem Behälter herausnehmbar und wieder einsetzbar ist.

13. Dusche nach einem der vorangehenden Ansprüche, **dadurch gekennzeichnet, daß** der Ventilkörper (20) in seitlichen Lagerlappen (32) des Behälters (1) gelagert ist, die C-förmige, über mehr als 180° Umfangswinkel verlaufende Lagerstege (36) aufweisen.

14. Dusche nach Anspruch 13, **dadurch gekennzeichnet, daß** die Lagerlappen (32) an Seitenwänden (16) des Behälters ausgebildet sind, zwischen denen Ventilkörper (20) und Rohr (22) in Belüftungsstellung aufnehmbar sind.

15. Dusche nach einem der vorangehenden Ansprüche, **dadurch gekennzeichnet, daß** die Standflächen (8) durch freie Endabschnitte von Seitenwänden (16) des Behälters ( 1) gebildet werden.

16. Dusche nach einem der vorangehenden Ansprüche, **dadurch gekennzeichnet, daß** das Rohr (22) einteilig mit dem Ventilkörper (20) ausgebildet oder auf diesen aufsteckbar ausgebildet ist.

17. Dusche nach einem der vorangehenden Ansprüche, **dadurch gekennzeichnet, daß** der Behälter (1) mit einem dicht schließenden Deckel (2) versehen ist, der eine Dosierungsöffnung (6) aufweist.

18. Dusche nach Anspruch 17, **dadurch gekennzeichnet, daß** der Deckel (2) so auf dem Behälter (1) gehalten ist, daß er auch bei Innendruck dicht schließt.

## Claims

1. A nasal douche, comprising a container (1) for rinsing liquid, said container having an exit opening (14), a valve body (20) held movably in the region of the exit opening (4) and having a tube (22) attached to said valve body, wherein different pivoted positions of the valve body (20) in conjunction with the exit opening (14) fix an open position (Fig. 2) in which the tube (22) is connected to the container (1), and a closed position (Fig. 1), in which the container (1) is closed, **characterised in that** the valve body (20) is pivotable into an aerating position (Fig. 3) which differs from the open and closed positions and in which the container can be completely emptied and aerated.

2. A douche according to claim 1, **characterised in that** the container has an outer contour which engages over or covers the valve body (20) and tube (22) in the aerating position, the said outer contour being formed with support surfaces (18) on which the douche can be set down.

3. A douche according to claim 1 or 2, **characterised in that** the valve body (20) comprises a complete emptying opening (42) which in the aerating position communicates with the exit opening (14).

4. A douche according to any one of claims 1 to 3, **characterised in that** the valve body (20) has a curved sealing surface (24) which co-operates with the exit opening (14) in the open and closed positions, while in the open position a connecting duct (26) connected to the tube (22) and leading into the sealing surface (24) communicates with the exit opening (14).

5. A douche according to claim 4, **characterised in that** the outside of the container has an abutment surface (25) curved to correspond to the sealing surface (24) in the region of the dispensing opening (14).

6. A douche according to any one of the preceding claims, **characterised in that** the valve body (20) has associated detent elements (38) which in at least one position, particularly the closed and/or aerating position, co-operate with corresponding detent elements (40) of the container and secure the valve body (20) against unintentional pivoting.

7. A douche according to any one of the preceding claims, **characterised in that** the valve body (20) can be transferred from the closed position to the open position by pivoting through about 30° to about 60°, particularly about 45°.

8. A douche according to any one of the preceding claims, **characterised in that** the valve body (20) is of cylindrical construction and its end portions are provided with bearing attachments (21).

9. A douche according to any one of the preceding claims, **characterised in that** the valve body (20) has cylindrical bearing attachments (21) to be received in corresponding bearing recesses (34, 36) of the container (1).

10. A douche according to claim 9, **characterised in that** the bearing attachments (21) and bearing recesses (34, 36) are of asymmetrical construction.

11. A douche according to any one of claims 8 to 10, **characterised in that** the complete emptying opening (42) is formed as a flattening of the cylindrical valve body (20).

12. A douche according to any one of the preceding claims, **characterised in that** the valve body can be removed from and re-inserted in the container non-destructively.

13. A douche according to any one of the preceding claims, **characterised in that** the valve body (20) is mounted in lateral bearing lugs (32) of the container (1) which have C-shaped bearing webs (36) extending over a peripheral angle of more than 180°.

14. A douche according to claim 13, **characterised in that** the bearing lugs (32) are formed on side walls (16) of the container, between which the valve body (20) and tube (22) can be received in the aerating position.

15. A douche according to any one of the preceding claims, **characterised in that** the support surfaces (8) are formed by free end portions of side walls (16) of the container (1).

16. A douche according to any one of the preceding claims, **characterised in that** the tube (22) is formed in one piece with the valve body (20) or is constructed to be pushed on to the latter.

17. A douche according to any one of the preceding claims, **characterised in that** the container (1) is provided with a sealing lid (2) having a dispensing opening (6).

18. A douche according to claim 17, **characterised in that** the lid (2) is so held on the container (1) that it seals even in the event of internal pressure.

## Revendications

1. Douche nasale comprenant un récipient (1) pour un liquide de rinçage, présentant une ouverture de sortie (14), avec un corps de soupape (20) maintenu de façon mobile dans la zone de l'ouverture de la sortie (14), avec un tube (22) appliqué sur celui-ci, sachant que des positions de pivotement différentes du corps de soupape (20), en coopération avec l'ouverture de sortie (14), fixent une position d'ouverture (fig. 2), à laquelle le tube (22) est relié au récipient (1), et une position de fermeture (fig. 1), à laquelle le récipient (1) est fermé, **caractérisée en ce que** le corps de soupape (1) est susceptible de pivoter en une position d'aération (fig. 3) différente de la position d'ouverture et de fermeture, position d'aération à laquelle le récipient peut être vidé de son reste et aéré.

2. Douche selon la revendication 1, **caractérisée en ce que** le récipient présente un contour extérieur entourant ou recouvrant le corps de soupape (20) et un tube (22) à la position d'aération, contour extérieur sur lequel sont réalisées des surfaces de pose (18) sur lesquelles une douche peut être posée.

3. Douche selon la revendication 1 ou 2, **caractérisée en ce que** le corps de soupape (20) présente un évidement de vidage du reste (42) communiquant avec l'ouverture de sortie (14) lorsqu'on est en position d'aération.

4. Douche selon la revendication 1 à 3, **caractérisée en ce que** le corps de soupape (20) présente une surface d'étanchéité (24) incurvée qui, en position d'ouverture et de fermeture, coopère avec une ouverture de sortie (14), sachant que, en position d'ouverture, un canal de liaison (26), relié au tube (22) et débouchant dans la face d'étanchéité (24), communique avec l'ouverture de sortie (14).

5. Douche selon la revendication 4, **caractérisée en ce que** le récipient présente extérieurement dans la zone de l'ouverture de distribution (4) une surface d'appui (25) incurvée de manière correspondante à la face d'étanchéité (24).

6. Douche selon l'une des revendications précédentes, **caractérisée en ce qu'**au corps de soupape (20) sont associés des éléments d'encliquetage (38) qui, en au moins une position, en particulier la position de fermeture et/ou d'ouverture, coopèrent avec des éléments d'encliquetage (40) correspondants du récipient et assurent le corps de soupape (20) contre tout pivotement intempestif.

7. Douche selon l'une des revendications précédentes, **caractérisée en ce que** le corps de soupape (20) est susceptible de passer par pivotement d'un angle d'environ 30° à environ 60° en particulier d'environ 45° de la position de fermeture à la position d'ouverture.

8. Douche selon l'une des revendications précédentes, **caractérisée en ce que** le corps de soupape (20) est conformé en cylindre et est muni d'appendices de tourillonnement (21) sur ses tronçons d'extrémité.

9. Douche selon l'une des revendications précédentes, **caractérisée en ce que** le corps de soupape (20) présente des appendices de tourillonnement (21) cylindriques, devant être logés dans des évidements de tourillonnement (34, 36) correspondants du récipient (1).

10. Douche selon la revendication 9, **caractérisée en ce que** les appendices de tourillonnement (21) et les évidements de tourillonnement (34, 36) sont de forme asymétrique.

11. Douche selon l'une des revendications 8 à 10, **caractérisée en ce que** l'évidement de vidage du reste (42) est réalisé sous la forme de méplat du corps de soupape (20) cylindrique.

12. Douche selon l'une des revendications précédentes, **caractérisée en ce que** le corps de soupape peut être extrait et réinséré dans le récipient sans provoquer de destruction.

13. Douche selon l'une des revendications précédentes, **caractérisée en ce que** le corps de soupape (20) est monté dans des languettes de palier (32) latérales du récipient (1), qui présente des nervures de tourillonnement (36) en forme de C s'étendant sur plus de 180° d'angle périphérique.

14. Douche selon la revendication 13, **caractérisée en ce que** les languettes de tourillonnement (32) sont réalisées sur des parois latérales (26) du récipient entre lesquelles peuvent être logées en position d'aération le corps de soupape (20) et le tube (22).

15. Douche selon l'une des revendications précédentes, **caractérisée en ce que** les surfaces de pose (8) sont formées par des tronçons d'extrémité libres de parois latérales (16) du récipient (1).

16. Douche selon l'une des revendications précédentes, **caractérisée en ce que** le tube (22) est réalisé d'une seule partie avec le corps de soupape (20) ou bien peut être enfiché sur celui-ci.

17. Douche selon l'une des revendications précédentes, **caractérisée en ce que** le récipient (1) est muni d'un couvercle (2) fermant de façon étanche, présentant une ouverture de dosage (6).

18. Douche selon la revendication 17, **caractérisée en ce que** le couvercle (2) est maintenu sur le récipient (1) de manière à ce qu'il ferme de façon étanche, même en cas de pression intérieure.
